# EUROPEAN PATENT SPECIFICATION

(11) **EP 2 983 684 B1**
(45) Date of publication and mention of the grant of the patent: **06.09.2017**
(21) Application number: 14730582.5
(22) Date of filing: 11.04.2014
(51) Int. Cl.: A61K 36/235, A61K 36/53, A61K 36/534, A61K 36/537, A61K 36/61, A61K 36/54, A61P 29/00

(54) **ESSENTIAL OIL AND ALOE FOR TREATMENT AND PROPHYLAXIS OF INFLAMMATIONS CAUSED BY DEMODEX, ESPECIALLY MARGINAL BLEPHARITIS, A PHARMACEUTICAL COMPOSITION CONTAINING ESSENTIAL OIL AND/OR ALOE AND THE USE OF ESSENTIAL OIL AND ALOE AND THEIR COMPOSITIONS FOR THE PRODUCTION OF A PREPARATION USED IN TREATMENT AND PROPHYLAXIS OF THE MENTIONED INFLAMMATIONS**
ÄTHERISCHES ÖL UND ALOE ZUR BEHANDLUNG UND PROPHYLAXE VON DURCH DEMODEX VERURSACHTEN ENTZÜNDUNGEN, INSBESONDERE MARGINALER BLEPHARITIS, EINE PHARMAZEUTISCHE ZUSAMMENSETZUNG MIT DEM ÄTHERISCHEM ÖL UND/ODER ALOE UND VERWENDUNG DES ÄTHERISCHEN ÖLS UND ALOE UND DEREN ZUSAMMENSETZUNGEN ZUR HERSTELLUNG EINES IN DER BEHANDLUNG UND PROPHYLAXE DER GENANNTEN ENTZÜNDUNGEN VERWENDETEN PRÄPARATS
HUILE ESSENTIELLE ET ALOÈS POUR LE TRAITEMENT ET LA PROPHYLAXIE D'INFLAMMATIONS PROVOQUÉES PAR DEMODEX, NOTAMMENT LA BLÉPHARITE MARGINALE, COMPOSITION PHARMACEUTIQUE CONTENANT UNE HUILE ESSENTIELLE ET/OU DE L'ALOÈS ET UTILISATION DE L'HUILE ESSENTIELLE ET DE L'ALOÈS ET DE LEURS COMPOSITIONS POUR LA PRODUCTION D'UNE PRÉPARATION UTILISÉE DANS LE TRAITEMENT ET LA PROPHYLAXIE DES INFLAMMATIONS MENTIONNÉES

(30) Priority: 11.04.2013 PL 40350913
(43) Date of publication of application: 17.02.2016
(73) Proprietor: Ofta Sp. z.o.o., 03-667 Warszawa (PL)
(72) Inventor: OSEKA, Maciej, PL-05-462 Duchnów (PL); ROMAN, Beata, PL-02-759 Warszawa (PL); JAREMKO-PIEKARSKA, Emilia, PL-05-500 Piaseczno (PL); SEDZIKOWSKA, Aleksandra, PL-02-798 Warszawa (PL)
(74) Representative: Dargiewicz, Joanna
(86) International application number: PCT/IB2014/060668
(87) International publication number: WO 2014/167552

(56) References cited:
- MORSY TOSSON A ET AL: "Eucalyptus globulus (camphor oil) in the treatment of human demodicidosis.", JOURNAL OF THE EGYPTIAN SOCIETY OF PARASITOLOGY DEC 2002, vol. 32, no. 3, December 2002 (2002-12), pages 797-803, XP009179224, ISSN: 1110-0583
- DATABASE WPI Week 201353 Thomson Scientific, London, GB; AN 2013-H76539 XP002727525, & CN 102 921 051 A (YIN Y) 13 February 2013 (2013-02-13)
- DATABASE WPI Week 201048 Thomson Scientific, London, GB; AN 2010-B42890 XP002727526, & KR 2010 0006808 A (KIM M) 22 January 2010 (2010-01-22)
- DATABASE WPI Week 201129 Thomson Scientific, London, GB; AN 2011-A32712 XP002727527, & CN 101 904 756 A (YIN Y) 8 December 2010 (2010-12-08)
- ZHAO YA-E ET AL: "The pesticidal effect and mechanism of peppermint oil in vitro against Demodex", CHINESE BULLETIN OF ENTOMOLOGY, vol. 44, no. 1, January 2007 (2007-01), pages 74-77, XP009179242, ISSN: 0452-8255
- SILVA JEANE ET AL: "Analgesic and anti-inflammatory effects of essential oils of Eucalyptus", JOURNAL OF ETHNOPHARMACOLOGY, ELSEVIER IRELAND LTD, IE, vol. 89, no. 2-3, 1 December 2003 (2003-12-01), pages 277-283, XP002422054, ISSN: 0378-8741, DOI: 10.1016/J.JEP.2003.09.007
- DATABASE WPI Week 200847 Thomson Scientific, London, GB; AN 2008-H42286 XP002727528, & KR 100 668 878 B1 (KANG M Y) 12 January 2007 (2007-01-12)
- DATABASE WPI Week 200522 Thomson Scientific, London, GB; AN 2005-206101 XP002727529, & JP 2005 060320 A (NIPPON NETWORK KK) 10 March 2005 (2005-03-10)
- WILLIAMSON ET AL: "An investigation and comparison of the bioactivity of selected essential oils on human lice and house dust mites", FITOTERAPIA, IDB HOLDING, MILAN, IT, vol. 78, no. 7-8, 25 October 2007 (2007-10-25), pages 521-525, XP022314127, ISSN: 0367-326X, DOI: 10.1016/J.FITOTE.2007.06.001
- CZEPITA DAMIAN ET AL: "Demodex folliculorum and Demodex brevis as a cause of chronic marginal blepharitis.", ANNALES ACADEMIAE MEDICAE STETINENSIS 2007, vol. 53, no. 1, 2007, page 63, XP009179230, ISSN: 1427-440X

## Description

The subject of the patent is a pharmaceutical composition containing as active biocidal substances essential sage oil, and aloe for use in a method of treatment and prophylaxis of inflammation caused by Demodex, especially marginal blepharitis.

Demodex is an arachnid, a member of the mite family, it is one of the best-known ectoparasites - Demodicidae family, among which two species parasitize humans: Demodex folliculorum and Demodex brevis. The occurrence of Demodex is extremely common. In tests carried out on different populations it was found that the percentage of people infected with Demodex ranges between 15 and 80%. The proportion of people infected with Demodex rises with the increase in age, which is probably connected with decreased immunity in elderly people. Some sources report 84% in the population of people at the age of 60 and even 100% in people above the age of 70. In children it occurs very rarely and it mainly affects children with immunity disorders. The scarcity of occurrence in children may result from small amounts of sebum secreted by sebaceous and tarsal glands.

Infections caused by mites such as Demodex are spread by contact transmission and most probably by dust which may contain eggs. Humans cannot contract the infections from animals because Demodex is species-specific.

Demodicidae are ectoparasites and both species live in hair follicles and sebaceous glands on the skin of the face, where they feed on lipids and products of sebaceous secretion. The ones occurring in greatest numbers are located in the region of the nose, around the eyes, on the forehead and chin, sometimes they can parasitize other parts of the body e.g. the skin of the hands and feet.

Tests carried out in the last 20 years indicate that the cause of marginal blepharitis may be the fact that Demodex live in the hair follicles of eyelashes and probably in the openings of meibomian glands, where they feed on their secretion. By blocking the openings of sebaceous glands ducts Demodex cause changes in the eye's tear film resulting from a lipid layer deficiency and inflammations caused by the accumulation of secretion in the glands. Demodex living in sebaceous glands of the skin may lead to an inflammation in the form of acne rosacea.

### Marginal blepharitis caused by Demodex

Marginal blepharitis is a big clinical problem in ophthalmology practice. Demodex, living in the hair follicles of eyelashes, causes persistent, chronic marginal blepharitis and blepharoconjunctivitis.

Marginal blepharitis involves a dysfunction of eyelid glands. There are two types of marginal blepharitis:
- anterior - involving mainly a dysfunction of sebaceous glands opening to the hair follicles of eyelashes
- posterior - involving a plugging of meibomian glands orifices.

Demodex, by moving in the follicle, mechanically irritate the papilla and the hair bulb and their metabolic waste causes chemical irritation and swelling of the papilla. The objective symptoms of marginal blepharitis are: thickening and redness of the eyelid, a discharge on the surface of the eyelid margin and often symptoms of a dry eye syndrome. The subjective symptoms of marginal blepharitis are: severe itching, pain and a gritty sensation under the eyelids.

Marginal blepharitis is usually chronic. Remission periods are interrupted by periods when the symptoms become more acute, exacerbated.

In the course of the parasite's growing in the follicle, the base of the eyelash may become dislocated and misdirected. One of the symptoms of infection is excessive loss of eyelashes.

Sometimes complications arise in the form of a bacterial inflammation of the hair follicle caused by staphylococcus - hordeolum or chalazia.

The medical condition caused by the presence of Demodex is called demodicosis. The following processes are responsible for the symptoms of demodicosis: blockage of follicles and leading out tubules of sebaceous glands, reactive hyperkeratinization and epithelial hyperplasia, mechanical transmission of bacteria and/or fungi (secondary bacterial/mycotic infection), the host's inflammatory reaction to the presence of parasite's chitin, stimulation of the host's humoral responses and cell-mediated immunological reaction caused by the presence of mites and their excretion.

The condition is frequently misdiagnosed as an allergic inflammation or a bacterial or mycotic infection. The process of changes caused by Demodex may display no clear pathological changes. In some patients the symptoms are not very distinctive. Patients complain mainly about persistent itching, burning sensation and redness of the eyelids.

### Long-term marginal blepharitis

Treated and untreated long-term marginal blepharitis may lead to the deformation of the eyelid margins and conditions resulting from it such as the damage of the cornea, lagophthalmos, drying out of the eye surface. Additionally, there may arise complications connected with long-term use of medicine, such as: viral corneitis, bacterial inflammation of the surface of the eye caused by drug-resistant bacteria and the damage of the eye's tear film, leading to excessive drying of the surface of the eye.

### Currently used treatment of marginal blepharitis

Currently used treatment of marginal blepharitis mainly comes down to symptomatic treatment, that is easing of the inflammation of the eyelid margins. The medicine used in such cases are mainly steroid medications in the form of eye ointments or non-steroidal anti-inflammatory drugs in the form of eye drops. Additionally, topical antibiotics in the form of ointment or drops are used, or combination antibiotic-steroid drugs in the form of ointment or drops.

The effectiveness of treatment with substances such as: metronidazole ointment, silver nitrate, or mechanical removal of lid margin debris is questionable. The only preparation showing therapeutic effect was mercuric oxide ointment, which for reasons of safety and because of the binding regulations cannot be used.

Yet, in most cases the troublesome symptoms return after inflammatory treatment.

Moreover, during the currently used treatment, especially with steroid drugs, one needs to have patience because the fight against this parasite is not easy. The treatment should be started not earlier than in autumn, when the sun is no longer so high.

Ignoring this recommendation can cause discolouration of the skin because the drugs against Demodex sensitise the skin tosunlight. The second condition is the necessity to use disposable towels by all the household members during the treatment, as well as boil the towels that have been used thus far. Failure to meet the conditions may cause secondary infections with the parasite.

That is why there is still an unfulfilled need for developing a remedy for the treatment and prophylaxis of inflammations caused by Demodex, especially around the eyes, in particular a preparation for the treatment and prophylaxis of marginal blepharitis.

The use of essential oils as medicinal and cosmetic products is well known. And thus, the international patent application WO1995032723A1 disclosed a fitotherapeutic remedy for the treatment of skin damage and lesions, which consists of the extract of ingredients which can be extracted from sage (*Salvia officinalis*)*,* ribwort plantain (*Plantago lanceolata*)*,* broadleaf plantain *(Plantago major)* and mistletoe *(Viscum album*)*.* The international patent application WO 200016752 A2 described a method of treatment of an epithelium condition in a patient, such as inflammation, an insect bite, an allergic reaction, sunburn, eczema, swelling, acne, dry skin, oily skin, bad odour, chafe, cut and bruising, which makes use of a device for cosmetic aromatherapy treatment of a patient with an aromatherapy composition containing essential oils and a polymeric matrix.

The use of essential oils was also described in the international patent applications WO 200905861 A1, WO 2011109139 A2, WO 2012050763 A2 and in the Japanese patent application JP 2012111727 A.

The international patent application WO 200912679 8A1 disclosed a composition of Salvia hispanica seed extract and a method of preparing it, which can be used to alleviate or prevent illnesses, such as cardiovascular diseases, arthritis pain, inflammation, platelet aggregation or to treat the dry eye syndrome, premenstrual symptoms or to modify the immune response in people or animals.

The international patent application WO 2010141591 A1 refers to the composition of an ophthalmic emulsion useful in the treatment of eye diseases including the dry eye syndrome, which in one realisation contains oil with omega-3 acids, which comes from a botanic source such as Salvia hispanica among other things. The emulsion according to the patent can be used to deliver different medicinal products in the form of eye drops in order to treat different eye diseases such as the dry eye syndrome, glaucoma, conjunctivitis, marginal blepharitis, allergies and infections.

However, in the European patent description EP 1185178 B1 a pharmaceutical composition containing plant essential oils and extracts in a gel carrier for use in fighting against insects, especially parasitic insects like ectoparasites such as lice, mites, and fleas.

Nonetheless, none of the found documents disclosed the use of essential sage oil in the treatment and prophylaxis of inflammations caused by Demodex, especially marginal blepharitis, nor did they disclose the use of such oil for the production of a drug for use in the treatment and prophylaxis of the listed inflammations.

Therefore, the purpose of this invention was to find an effective remedy for the treatment and prophylaxis of inflammations caused by Demodex, especially around the eyes, in particular a remedy for the treatment and prophylaxis of marginal blepharitis. Thorough testing was conducted in the search for substances that are effective in fighting against Demodex, which will be able for use in the treatment and prophylaxis of inflammations, especially marginal blepharitis. Unexpectedly, it was found that natural essential sage oil and aloe display a biocidal activity towards Demodex, preventing and curing inflammations caused by these organisms.

And thus, in one aspect the subject of the disclosure is an essential oil selected from the group including: eucalyptus oil, camphor oil, lavender oil, fennel oil, sage oil, mint oil for use in the treatment and prophylaxis of inflammations caused by Demodex.

In particular, the subject of this disclosure is essential oil for the use in which the inflammation caused by Demodex is marginal blepharitis.

Also disclosed is sage essential oil, especially natural sage essential oil, such as *Salvia hispanica* oil, common sage *Salvia officinalis* oil or other species including *Salvia triloba (fruticosa), Salvia lavandulifolia* and *Salvia sclarea.*

In another aspect the disclosure describes the use of an essential oil selected from the group including: eucalyptus oil, camphor oil, lavender oil, fennel oil, sage oil, mint oil and aloe for the production of a preparation for use in the treatment and prophylaxis of inflammations caused by Demodex.

In particular, the disclosure describes the use in which the inflammation caused by Demodex is marginal blepharitis.

In particular, the disclosure describes the use in which the essential oil is sage essential oil, especially natural sage essential oil, such as *Salvia hispanica* oil, common sage *Salvia officinalis* oil or other species including *Salvia triloba (fruticosa), Salvia lavandulifolia* and *Salvia sclarea.*

In another aspect, the subject of this disclosure is aloe for the use for the treatment and prophylaxis of inflammations caused by Demodex.

In particular, the disclosure describes aloe for the use in which the inflammation caused by Demodex is marginal blepharitis.

In particular, the disclosure describes aloe selected from the group including: *Aloe vera, Aloe ferox* and *Aloe arborescens* Mill.

In another aspect the disclosure refers to the use of aloe for the production of a preparation for the use in the treatment and prophylaxis of inflammations caused by Demodex.

In particular, the disclosure describes the use in which the inflammation caused by Demodex is marginal blepharitis.

In particular, the disclosure describes the use in which aloe is selected from the group including: *Aloe vera, Aloe ferox* and *Aloe arborescens* Mill.

The subject of this invention is a pharmaceutical composition, which as an active biocidal ingredient contains sage oil, and aloe for the use in the treatment and prophylaxis of inflammations caused by Demodex.

Preferably, the subject of this invention is a composition for the use in which the inflammation caused by Demodex is marginal blepharitis.

A composition is also disclosed, which contains water; at least one essential oil selected from the group including: eucalyptus oil, camphor oil, lavender oil, fennel oil, sage oil, mint oil and/or aloe, emulsifier/thickening agent; emulsion stabilizer and emollient.

Preferably, the disclosed composition contains water in the amount of 10-90% by weight, aloe in the amount of 0,01-100% by weight (adjusted to 1:1 depending on the degree of concentration of the raw material) and/or at least one essential oil selected from the group including: eucalyptus oil, camphor oil, lavender oil, fennel oil, sage oil and mint oil in the amount of 0,1-80% by weight, emulsifier/thickening agent in the amount of 0,5-15% by weight, emulsion stabilizer in the amount of 0-15% by weight and emollient in the amount of 0-25% by weight.

Preferably, the composition according to the invention contains fatty acids esters as an emulsifier/thickening agent, acrylic polymers, polysaccharides, cellulose thickeners as emulsion stabilizers, fatty alcohols, fatty acids, esters, triglycerides, ceramides, waxes, vegetable oils, squalanes, mineral oils, silicones and the like as emollients.

Preferably, the composition according to the invention additionally contains at least one agent from among the group including a preservative, moisturizer, demulcent and complexing agent.

Preferably, the composition according to the invention contains an emollient; sage oil, and aloe and a thickening agent.

Preferably, the composition according to the invention contains an emollient in the amount of 20-80% by weight, aloe in the amount of 0,01-100% by weight (adjusted to 1:1 depending on the degree of concentration of the raw material) and essential oil selected from sage oil in the amount of 0,1-80% by weight; a thickening agent in the amount of 1-45% by weight.

Preferably, the composition according to the patent contains fatty alcohols, fatty acids, esters, triglycerides, ceramides, waxes, vegetable oils, squalanes, mineral oils, silicones as emollients, and it contains waxes, dextrins as thickening agents.

Preferably, the composition according to the patent additionally contains at least one agent from among the group including an antioxidant, moisturizer and demulcent.

Preferably, the composition according to the patent contains sage essential oil as an active biocidal ingredient, which is especially natural sage essential oil, such as *Salvia hispanica* oil, common sage *Salvia officinalis* oil or other species including *Salvia triloba (fruticosa), Salvia lavandulifolia* and *Salvia sclarea.*

Preferably, the composition according to the invention contains aloe as an active biocidal ingredient, selected from the group consisting of *Aloe vera, Aloe ferox* and and *Aloe arborescens* Mill.

Preferably, the composition according to the invention is for topical application in the form of emulsion, cream, gel, ointment, dressing, paste, foam, plaster, spray or solution.

Thanks to their unexpected biocidal properties against Demodex, essential oils such as eucalyptus oil, camphor oil, lavender oil, fennel oil, sage oil, mint oil and aloe can be used in the treatment and prophylaxis of inflammations caused by Demodex, especially marginal blepharitis, in the form of a pharmaceutical composition containing such an essential oil and/or aloe. Essential sage oil and aloe for the use according to this invention, taking into account the specificity of the treated condition, preferably can be administered in the form of preparations for topical application such as emulsion, cream, gel, ointment, dressing, paste, foam, plaster, spray or solution. These preparations can be applied on the eyelid for the purpose of treatment and prophylaxis of inflammations, especially marginal blepharitis, as well as e.g. topically, on the skin in other areas for the purpose of treatment and prophylaxis of inflammations, e.g. acne inflammations, especially those caused by Demodex. Both water and lipid formulations can be used in the preparations, whose frame formulations are presented in the examples below.

The way the preparation is used according to the invention is also essential, which takes into account the life cycle of Demodex, which lasts approximately 2-4 weeks, as well as its daily activity. The parasite is active mainly at night. Sunrays paralyse Demodex, forcing it to move back into deeper layers of skin. The developed dosage scheme includes topical application of the preparation according to the patent once a day, especially at night-time, for the period of approximately 2 weeks, then a 2-week break and another period of using the preparation for the next 2 weeks.

The present invention will be illustrated with the following examples.

### EXAMPLES

### Example 1

### In vitro experiments

As a result of the carried out research into the use of different substances, which may have biocidal activity against Demodex it turned out that essential oil such as eucalyptus oil, camphor oil, lavender oil, fennel oil, sage oil and mint oil show very strong biocidal activity against these organisms.

The experiments were conducted in the following way:
Freshly obtained eyelashes were placed on a microscopic slide with a teardrop, onto which solutions of drugs and substances of different concentrations were sprinkled. The whole of it was placed in a humidity chamber to avoid drying of the preparation. Mobility of Demodex was monitored under the microscope at different time intervals.

As a control group Demodex was placed in Ringer's solution and it was monitored how long they survived in this solution.

The influence of the following was tested:
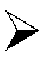 paraffin oil
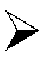 liquid silicone (Polydimethylsiloxane 4% Cyclopentasiloxane 86%)
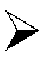 liquid silicone (Polydimethylsiloxane) of sea buckthorn oil 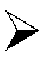 aloe juice
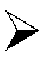 natural essential oils: sage, mint, camphor, fennel, eucalyptus and lavender
The results of the tests are presented in table 1 below.

**Table 1**

| **AGENT:** | **Average length of survival** | **Minimum length of survival** | **Maximum length of survival** |
|---|---|---|---|
| Ringer's solution | **92 hours** | 35 hours | 156 hours |
| paraffin oil | **120 hours** | 80 hours | 148 hours |
| liquid silicone (Polydimethylsiloxane 4% Cyclopentasiloxane 86%) | **100 hours** | 52 hours | 168 hours |
| liquid silicone (Polydimethylsiloxane) | **55 hours** | 40 hours | 90 hours |
| sea buckthorn oil | **72 hours** | 20 hours | 85 hours |
| aloe | **9 hours** | 2 hours | 11 hours |
| eucalyptus oil | **23 minutes** | 19 minutes | 30 minutes |
| camphor oil | **16,5 minutes** | 11 minutes | 27 minutes |
| lavender oil | **14 minutes** | 5 minutes | 18 minutes |
| fennel oil | **27,5 minutes** | 25 minutes | 37 minutes |
| sage oil | **7 minutes** | 2 minutes | 14 minutes |
| sage oil 50% | **32 minutes** | 30 minutes | 45 minutes |
| sage oil 25% | **85 minutes** | 31 minutes | 150 minutes |
| sage oil 12,5% | **119 minutes** | 175 minutes | 61 minutes |
| sage oil 10% (with triglycerides of caprinic and caprilic acids) | **36 hours** | 30 hours | 40 hours |
| sage oil 5% (with triglycerides of caprinic and caprilic acids) | **40 hours** | 20 hours | 60 hours |
| sage oil 0,5% (with triglycerides of caprinic and caprilic acids) | **80 hours** | 72 hours | 96 hours |
| mint oil | **10,5 minutes** | 6 minutes | 19 minutes |
| mint oil 50% ( with triglycerides of caprinic and caprilic acids) | **43 minutes** | 28 minutes | 70 minutes |
| sage oil 25% + mint oil 50% (with triglycerides of caprinic and caprilic acids) | **14 minutes** | 7 minutes | 18 minutes |
| sage oil 10% + mint oil 50% (with triglycerides of caprinic and caprilic acids) | **15 minutes** | 7 minutes | 21 minutes |
| sage oil 5% + mint oil 50% + camphor oil 20% (with triglycerides of caprinic and caprilic acids) | **7 minutes** | 3 minutes | 8 minutes |
| sage oil 5% + mint oil 50% + fennel oil 20% (with triglycerides of caprinic and caprilic acids) | **7 minutes** | 4 minutes | 9 minutes |
| sage oil 5% + mint oil 50% + lavender oil 10% (with triglycerides of caprinic and caprilic acids) | **8 minutes** | 3 minutes | 10 minutes |
| sage oil 5% + oil solution of aloe (with triglycerides of caprinic and caprilic acids) | **8 hours** | 2 hours | 11 hours |
| sage oil 5% + aloe | **2 hours** | 30 minutes | 5 hours |
| sage oil 0,5% + aloe | **8 hours** | 2 hours | 10 hours |
| sage oil 0,1% + aloe | **9 hours** | 2 hours | 11 hours |

The first tested substances in which biocidal activity against Demodex was established were essential oils such as eucalyptus oil, camphor oil, lavender oil, fennel oil, sage oil, and mint oil used alone or in mixtures. That is why it was initially stated that the optimal preparation for the treatment and prophylaxis of inflammations, especially those caused by Demodex, would be a preparation containing selected oils or their mixtures.

However, it turned out that essential oils irritate the skin of eyelids when used in higher concentrations. Therefore, a further search was carried out for an additional substance with a high safety profile. Unexpectedly, it turned out that aloe, a commonly used substance showing the desirable properties, also has biocidal properties against Demodex, thanks to which a composition containing aloe alone as an active ingredient, or a mixture of one of the above essential oils and aloe will be effective in the treatment and prophylaxis of inflammations, especially those caused by Demodex, in the intended time interval.

### Example 2

### Preparing pharmaceutical compositions

Taking into account above all the safety of using essential oils especially in the area around eyes, as well as improving the barrier function of skin, epidermis and properties supporting the immune response of skin infected by Demodex, a product was developed in which the ingredients with such a spectrum of activity were selected.

The use of aloe in the formula eliminates itching and burning which accompany inflammations.

D-panthenol (provitamin of B5) imparts elasticity to skin and reduces irritation/relieves irritation.

Hyaluronic acid together with Fucogel® create a film and ensure comprehensive moisturising of the irritated skin.

Because of its composition similar to the lipid layer of human skin, lanolin shows strong moisturising and softening properties and has a favourable influence on regenerative and repairing processes of damaged skin.

The developed product for specialist protection and eyelid care
- helps to remove the accumulated impurities/debris and discharge on eyelids and eyelid margins
- alleviates symptoms of irritation caused by different external factors (such as e.g. Demodex)
- moisturises the sensitive eyelid skin and stimulates regenerative processes
- eliminates discomfort connected with the drying and damage of epidermis

Moreover, as a result of the analysis of the in vitro tests results, where the effectiveness against Demodex and the safety profile were established and the fact that the preparation will be applied topically at night-time, compositions were developed which show activity in the period of 4-8 hours. A water solution of aloe 1:1 also shows effectiveness in this time frame. Concentrated aloe is used for production, most frequently at the degree of concentration 200:1.

In the case of preparations to be used on eyelids, the concentration of essential oils will be lower and will be 10% by weight.

However, in preparations for topical use in case of acne, the concentration of essential oils will be higher, it will be 80% by weight.

Devices used to prepare the formulation:
- mechanical mixer - IKA RW 20 D
- laboratory homogeniser - VELP OV 5
- general laboratory balance

Two frame formulations were developed, one containing water phase and oil phase, the second one is lipid-based.

If not indicated otherwise, the numbers given in percent (%) mean % by weight.

### Frame formulation no. 1

| Item number | Ingredient | Range |
|---|---|---|
| 1 | Water | 10-90% |
| 2 | Essential oil or a mixture of essential oils | 0,0-80% |
| 3 | Aloe (different species, different concentrations) | 0,0-100% (adjusted to1:1) |
| 4 | Emulsifier/Thickener | 0,5-15% |
| 5 | Emulsion stabilizer | 0-15% |
| 5 | Emollient (e.g. fatty alcohols, fatty acids, esters, triglycerides, ceramides, waxes, vegetable oils, squalanes, mineral oils, silicones) | 0-25% |

| | | |
|---|---|---|
| *The composition may contain aloe of different concentrations | | |

Additionally, the composition may contain any one of preserving agents, any one of moisturising, soothing agents, any complexing agent.

### Frame formulation no. 2

| Item number | Ingredient | Range |
|---|---|---|
| 1 | Emollient (fatty alcohols, fatty acids, esters, triglycerides, ceramides, waxes, vegetable oils, squalanes, mineral oils, silicones) | 5-80% |
| 2 | Essential oil or a mixture of essential oils | 0,5- 80% |
| 3 | Aloe (different species, different concentrations) | 0,0-100% (adjusted to 1:1) |
| 4 | Thickener | 1-45% |

| | | |
|---|---|---|
| *The composition may contain aloe of different concentrations | | |

Additionally, the composition may contain any one of antioxidants, any one of moisturising and soothing agents.

The technological process (prescriptions no. 2.1-2.2): The weighted-out amount of water was brought to the boil and cooled down to the temperature of approximately 80°C. Olivem 1000 (cetearyl alcohol and fatty acids of olive oil ester (Cetearyl Olivate), sorbitan and fatty acids of olive oil ester (Sorbitan Olivate)) was heated to the temperature of approximately 75°C (until completely molten) and added to the water phase. The whole was homogenized for a few minutes until a homogeneous mixture was obtained, then the process of slow cooling was started. At the temperature of approximately 40°C an essential oil was added and the whole was mixed until a homogeneous mixture was obtained. While continuing the cooling, hyaluronic acid (HA), Fucogel and Aloe Vera, D-panthenol and Dissolvine GL-47-S (Tetrasodium Glutamate Diacetate) were added, each time stirring until a homogeneous mixture was obtained. At the end the preservative Euxyl PE 9010 (2-phenoxyethanol and 3-[(2-ethylhexyl)oxy]-1,2-propanediol in the ratio 9:1) was added.

### 2.1

| Item number | Ingredient | % (by weight) |
|---|---|---|
| 1 | Water | up to 100 |
| 2 | Sage oil | 0,1 |
| 3 | Aloe Vera* | 0,5 |
| 4 | Olivem 1000 (INCI: Cetearyl Olivate, Sorbitan Olivate) | 5,5 |
| 5 | Fucogel 1.5P | 3,0 |
| 6 | HA (low molecular, sodium salt) | 0,5 |
| 7 | D-panthenol | 1,0 |
| 8 | Euxyl PE 9010 (2-phenoxyethanol , 3-[(2-ethylhexyl) oxy] -1,2- propanediol in the ratio 9:1) | 1,1 |
| 9 | Dissolvine GL-47-S (Tetrasodium glutamate diacetate) | 0,1 |

| | | |
|---|---|---|
| *Aloe Vera degree of concentration 200:1 | | |

### 2.2

| Item number | Ingredient | % |
|---|---|---|
| 1 | Water | up to 100 |
| 2 | Sage oil | 0,5 |
| 3 | Aloe Vera* | 0,5 |
| 4 | Olivem 1000(INCI:Cetearyl Olivate, Sorbitan Olivate) | 5,5 |
| 5 | Fucogel 1.5P | 3,0 |
| 6 | HA (low molecular, sodium salt) | 0,5 |
| 7 | D-panthenol | 1,0 |
| 8 | Euxyl PE 9010 (2-phenoxyethanol , 3- [(2-ethylhexyl)oxy] -1 2- propanediol, in the ratio 9:1) | 1,1 |
| 9 | Dissolvine GL-47-S (Tetrasodium glutamate diacetate) | 0,1 |

| | | |
|---|---|---|
| *Aloe Vera degree of concentration 200:1 | | |

The technological process (prescription no. 2.3): The weighted-out amount of water was brought to the boil and cooled down to the temperature of approximately 80°C. Olivem 1000 (cetearyl alcohol and fatty acids of olive oil ester (Cetearyl Olivate), sorbitan ester and fatty acids of olive oil (Sorbitan Olivate)) was heated to the temperature of approximately 75°C (until completely molten) and added to the water phase. The whole was homogenized for a few minutes until a homogeneous mixture was obtained, then the process of slow cooling was started. At the temperature of approximately 40°C an essential oil was added and the whole was mixed until a homogeneous mixture was obtained. While continuing the cooling, hyaluronic acid (HA), Fucogel and Aloe Vera, D-panthenol and Dissolvine GL-47-S (Tetrasodium Glutamate Diacetate) were added, each time stirring until a homogeneous mixture was obtained. At the end the preservative Euxyl PE 9010 (2-phenoxyethanol and 3-[(2-ethylhexyl)oxy]-1,2- propanediol) was added.

### 2.3

| Item number | Ingredient | % |
|---|---|---|
| 1 | Water | up to 100 |
| 2 | Aloe Vera* | 0,5 |
| 3 | Olivem 1000 (INCI: Cetearyl Olivate, Sorbitan Olivate) | 5,5 |
| 4 | Fucogel 1.5P | 3,0 |
| 5 | HA (low molecular, sodium salt) | 0,5 |
| 6 | D-panthenol | 1,0 |
| 7 | Euxyl PE 9010 (2-phenoxyethanol , 3-[(2-ethylhexyl) oxy] -1,2- propanediol in the ratio 9:1) | 1,1 |
| 8 | Dissolvine GL-47-S (Tetrasodium glutamate diacetate) | 0,1 |

### Aloe Vera degree of concentration 200:1

The technological process (prescription no. 4-16): The weighted-out amount of water was brought to the boil and cooled down to the temperature of approximately 80°C. Olivem 1000 (Cetearyl Olivate, Sorbitan Olivate) and the other ingredients of the oil phase Cetyl Palmitate, Sorbitan Palmitate, triglycerides of caprinic and caprilic acid were heated to the temperature of approximately 75°C (until a homogeneous mixture was obtained) and were added to the water phase. The whole was homogenized for a few minutes until a homogeneous mixture was obtained, and then the process of slow cooling was started. At the temperature of approximately 40°C an appropriate essential oil was added and the whole was stirred thoroughly. While continuing the stirring Acrylates/Acrylate C10-30 Alkyl Acrylate Crosspolymer was/were added, after obtaining a homogeneous mixture it was neutralized with TEA Triethanolamine. Next to the homogeneous mixture were added in turn HA, Fucogel and Aloe Vera, D-panthenol and tetrasodium glutamate diacetate, each time stirring the whole for a few minutes. At the end the preservative Euxyl PE 9010 (2-phenoxyethanol and 3-[(2-ethylhexyl)oxy]-1,2- propanediol) was added.

### 2.4

| Item number | Ingredient | % |
|---|---|---|
| 1 | Water | up to 100 |
| 2 | Sage oil | 2,0 |
| 3 | Aloe Vera* | 0,5 |
| 4 | Olivem 1000 (INCI: Cetearyl Olivate, Sorbitan Olivate) | 3,5 |
| 5 | Oliwax LC (INCI: Cetyl Palmitate, Sorbitan Palmitate, Sorbitan Olivate) | 2,0 |
| 6 | Fucogel 1.5P | 3,0 |
| 7 | HA (low molecular, sodium salt) | 0,5 |
| 8 | D-panthenol | 1,0 |
| 9 | Acrylates/C10-30 Alkyl Acrylate Crosspolymer | 0,2 |
| 10 | Triethanolamine | 0,23 |
| 11 | Euxyl PE 9010 (2-phenoxyethanol , 3- [(2-ethylhexyl)oxy] -1 2- propanediol | 1,1 |
| 12 | Dissolvine GL-47-S (Tetrasodium glutamate diacetate) | 0,1 |

| | | |
|---|---|---|
| *Aloe Vera degree of concentration 200:1 | | |

### 2.5

| Item number | Ingredient | % |
|---|---|---|
| 1 | Water | to 100 |
| 2 | Sage oil | 5,0 |
| 3 | Aloe Vera* | 0,5 |
| 4 | Olivem 1000(INCI: Cetearyl Olivate, Sorbitan Olivate) | 3,5 |
| 5 | Oliwax LC (INCI: Cetyl Palmitate, Sorbitan Palmitate, Sorbitan Olivate) | 2,5 |
| 6 | Triglycerides of caprinic and caprilic acid | 5,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,23 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 200:1 | | |

### 2.6

| Item number | Ingredient | % |
|---|---|---|
| 1 | Water | up to 100 |
| 2 | Sage oil | 10,0 |
| 3 | Aloe Vera* | 0,5 |
| 4 | Olivem 1000 (INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 200:1 | | |

### 2.7

| Item number | Ingredient | % |
|---|---|---|
| 1 | Water | up to 100 |
| 2 | Sage oil | 15,0 |
| 3 | Aloe Vera* | 0,5 |
| 4 | Olivem 1000(INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 200:1 | | |

### 2.8

| Item number | Ingredient | % |
|---|---|---|
| 1 | Water | up to 100 |
| 2 | Sage oil | 20,0 |
| 3 | Aloe Vera* | 0,5 |
| 4 | Olivem 1000 (INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 200:1 | | |

**2.9**

| Item number | Ingredient | % |
|---|---|---|
| 1 | Water | up to 100 |
| 2 | Sage oil | 10,0 |
| 3 | Aloe Vera* | 0,05 |
| 4 | Olivem 1000(INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy]-1 2-propanediol) | 1,1 |
| 13 | Dissolvine GL-47-S (Tetrasodium glutamate diacetate) | 0,1 |

| | | |
|---|---|---|
| *Aloe Vera degree of concentration 200:1 | | |

### 2.10

| Item number | Ingredient | % |
|---|---|---|
| 1 | Water | up to 100 |
| 2 | Sage oil | 10,0 |
| 3 | Aloe Vera* | 0,1 |
| 4 | Olivem 1000(INCI: INCI: Cetearyl Olivate, Sorbitan Olivate) | 4,5 |
| 5 | Oliwax LC (Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 200:1 | | |

### 2.11

| Item number | Ingredient | % |
|---|---|---|
| 1 | Water | up to 100 |
| 2 | Sage oil | 10,0 |
| 3 | Aloe Vera* | 5,0 |
| 4 | Olivem 1000(INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 1:1 | | |

### 2.12

| Item number | Ingredient | % |
|---|---|---|
| 1 | Water | up to 100 |
| 2 | Sage oil | 10,0 |
| 3 | Aloe Vera* | 5,0 |
| 4 | Olivem 1000(INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 1:1 | | |

### 2.13

| Item number | Ingredient | % |
|---|---|---|
| 1 | Water | up to 100 |
| 2 | Sage oil | 10,0 |
| 3 | Aloe Vera* | 2,5 |
| 4 | Olivem 1000(INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 1:1 | | |

### 2.14

| Item number | Ingredient | % |
|---|---|---|
| 1 | Water | up to 100 |
| 2 | Sage oil | 10,0 |
| 3 | Aloe Vera* | 1,5 |
| 4 | Olivem 1000(INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 1:1 | | |

### 2.15

| Item number | Ingredient | % |
|---|---|---|
| 1 | Water | up to 100 |
| 2 | Sage oil | 10,0 |
| 3 | Aloe Vera* | 0,5 |
| 4 | Olivem 1000(INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera o degree of concentration200:1 | | |

### 2.16

| Item number | Ingredient | % |
|---|---|---|
| 1 | Water | up to 100 |
| 2 | Sage oil | 10,0 |
| 3 | Aloe Vera* | 0,1 |
| 4 | Olivem 1000(INCI: Cetearyl Olivate, Sorbitan Olivate) | 4,5 |
| 5 | Oliwax LC (INCI: Cetyl Palmitate, Sorbitan Palmitate, Sorbitan Olivate) | 3,0 |
| 6 | Triglycerides of caprinic and caprilic acid | 3,0 |
| 7 | Fucogel 1.5P | 3,0 |
| 8 | HA (low molecular, sodium salt) | 0,5 |
| 9 | D-panthenol | 1,0 |
| 10 | Acrylates/C10-30 Alkyl Acrylate Crosspolymer | 0,2 |
| 11 | Triethanolamine | 0,25 |
| 12 | Euxyl PE 9010 (2-phenoxyethanol , 3- [(2-ethylhexyl)oxy] -1 2- propanediol) | 1,1 |
| 13 | Dissolvine GL-47-S (Tetrasodium glutamate diacetate) | 0,1 |

| | | |
|---|---|---|
| *Aloe Vera degree of concentration 1:1 | | |

The technological process (prescriptions no. 2.17-2.26): To the weighted-out amount of triglycerides of caprinic acid were added in turn Lanolin, white beeswax (Cera Alba), the whole was heated to the temperature of 75°C (until the ingredients were completely molten). The whole was stirred until a homogeneous mixture was obtained. Then the process of cooling was started and at the temperature below 40°C Aloe Ferox and the appropriate essential oil were added, each time stirring until a homogeneous mixture was obtained.
**2.17**

| Item number | Ingredient | % |
|---|---|---|
| 1 | Triglycerides of caprinic and caprilic acid | 45,0 |
| 2 | Sage oil | 20,0 |
| 3 | Aloe Ferox* | 5,0 |
| 4 | Cera Alba | 10,0 |
| 5 | Lanolin | 20,0 |

| | | |
|---|---|---|
| *Aloe Ferox degree of concentration 1:1 | | |

**2.18**

| Item number | Ingredient | % |
|---|---|---|
| 1 | Triglycerides of caprinic and caprilic acid | 50,0 |
| 2 | Sage oil | 15,0 |
| 3 | Aloe Ferox* | 5,0 |
| 4 | Cera Alba | 10,0 |
| 5 | Lanolin | 20,0 |

| | | |
|---|---|---|
| *Aloe Ferox degree of concentration 1:1 | | |

**2.19**

| Item number | Ingredient | % |
|---|---|---|
| 1 | Triglycerides of caprinic and caprilic acid | 50,0 |
| 2 | Sage oil | 10,0 |
| 3 | Aloe Ferox* | 5,0 |
| 4 | Cera Alba | 15 |
| 5 | Lanolin | 20 |

| | | |
|---|---|---|
| *Aloe Ferox degree of concentration 1:1 | | |

**2.20**

| Item number | Ingredient | % |
|---|---|---|
| 1 | Triglycerides of caprinic and caprilic acid | 55,0 |
| 2 | Sage oil | 5,0 |
| 3 | Aloe Ferox* | 5,0 |
| 4 | Cera Alba | 15,0 |
| 5 | Lanolin | 20,0 |

| | | |
|---|---|---|
| *Aloe Ferox degree of concentration 1:1 | | |

**2.21**

| Item number | Ingredient | % |
|---|---|---|
| 1 | Triglycerides of caprinic and caprilic acid | 57,5 |
| 2 | Sage oil | 2,5 |
| 3 | Aloe Ferox* | 5,0 |
| 4 | Cera Alba | 15,0 |
| 5 | Lanolin | 20,0 |

| | | |
|---|---|---|
| *Aloe Ferox degree of concentration 1:1 | | |

**2.22**

| Item number | Ingredient | % |
|---|---|---|
| 1 | Triglycerides of caprinic and caprilic acid | 59,0 |
| 2 | Sage oil | 1,0 |
| 3 | Aloe Ferox* | 5,0 |
| 4 | Cera Alba | 15,0 |
| 5 | Lanolin | 20 |

| | | |
|---|---|---|
| *Aloe Ferox degree of concentration 1:1 | | |

**2.23**

| Item number | Ingredient | % |
|---|---|---|
| 1 | Triglycerides of caprinic and caprilic acid | 42,5 |
| 2 | Sage oil | 5,0 |
| 3 | Aloe Ferox* | 2,5 |
| 4 | Cera Alba | 15,0 |
| 5 | Lanolin | 20,0 |

| | | |
|---|---|---|
| *Aloe Ferox degree of concentration 1:1 | | |

**2.24**

| Item number | Ingredient | % |
|---|---|---|
| 1 | Triglycerides of caprinic and caprilic acid | 59,0 |
| 2 | Sage oil | 5,0 |
| 3 | Aloe Ferox* | 1,0 |
| 4 | Cera Alba | 15,0 |
| 5 | Lanolin | 20,0 |

| | | |
|---|---|---|
| *Aloe Ferox degree of concentration 1:1 | | |

**2.25**

| Item number | Ingredient | % |
|---|---|---|
| 1 | Triglycerides of caprinic and caprilic acid | 59,5 |
| 2 | Sage oil | 5,0 |
| 3 | Aloe Ferox* | 0,5 |
| 4 | Cera Alba | 15,0 |
| 5 | Lanolin | 20,0 |

| | | |
|---|---|---|
| *Aloe Ferox degree of concentration 1:1 | | |

**2.26**

| Item number | Ingredient | % |
|---|---|---|
| 1 | Triglycerides of caprinic and caprilic acid | 59,9 |
| 2 | Sage oil | 5,0 |
| 3 | Aloe Ferox* | 0,1 |
| 4 | Cera Alba | 15,0 |
| 5 | Lanolin | 20,0 |

| | | |
|---|---|---|
| *Aloe Ferox degree of concentration 1:1 | | |

Technological process (prescription no. 2.27): To the weighted-out amount of triglycerides of caprinic acid were added in turn Lanolin, white beeswax (Cera Alba), the whole was heated to the temperature of 75°C (until the ingredients were completely molten). The whole was stirred until a homogeneous mixture was obtained. Then the process of cooling was started and at the temperature below 40°C the appropriate essential oil was added, the whole was stirred until a homogeneous mixture was obtained.
**2.27**

| Item number | Ingredient | % |
|---|---|---|
| 1 | Triglycerides of caprinic and caprilic acid | 45,0 |
| 2 | Sage oil | 20,0 |
| 3 | Cera Alba | 15,0 |
| 4 | Lanolin | 20,0 |

Technological process (prescription no. 2.28-2.37): To the weighted-out amount of triglycerides of caprilic and caprinic acid were added in turn Lanolin, Rheopearl KL2, the whole was heated to the temperature of 85°C (until the ingredients were completely molten). The whole was stirred until a homogeneous mixture was obtained. Then the process of cooling was started and at the temperature below 40°C Aloe Ferox and the appropriate essential oil were added, each time stirring until a homogeneous mixture was obtained.
**2.28**

| Item number | Ingredient | % |
|---|---|---|
| 1 | Triglycerides of caprinic and caprilic acid | 34,5 |
| 2 | Sage oil | 50,0 |
| 3 | Aloe Ferox* | 0,5 |
| 4 | Rheopearl KL2 (Dextrin palmitate) | 10,0 |
| 5 | Lanolin | 5,0 |

| | | |
|---|---|---|
| *Aloe Ferox degree of concentration 1:1 | | |

**2.29**

| Item number | Ingredient | % |
|---|---|---|
| 1 | Triglycerides of caprinic and caprilic acid | 34,5 |
| 2 | Mint oil | 50,0 |
| 3 | Aloe Ferox* | 0,5 |
| 4 | Rheopearl KL2 (Dextrin palmitate) | 10,0 |
| 5 | Lanolin | 5,0 |

| | | |
|---|---|---|
| *Aloe Ferox degree of concentration 1:1 | | |

**2.30**

| Item number | Ingredient | % |
|---|---|---|
| 1 | Triglycerides of caprinic and caprilic acid | 34,5 |
| 2 | Fennel oil | 50,0 |
| 3 | Aloe Ferox* | 0,5 |
| 4 | Rheopearl KL2 (Dextrin palmitate) | 10,0 |
| 5 | Lanolin | 5,0 |

| | | |
|---|---|---|
| *Aloe Ferox degree of concentration 1:1 | | |

**2.31**

| Item number | Ingredient | % |
|---|---|---|
| 1 | Triglycerides of caprinic and caprilic acid | 34,5 |
| 2 | Lavender oil | 50,0 |
| 3 | Aloe Ferox* | 0,5 |
| 4 | Rheopearl KL2 (Dextrin palmitate) | 10,0 |
| 5 | Lanolin | 5,0 |

| | | |
|---|---|---|
| *Aloe Ferox degree of concentration 1:1 | | |

**2.32**

| Item number | Ingredient | % |
|---|---|---|
| 1 | Triglycerides of caprinic and caprilic acid | 34,5 |
| 2 | Camphor oil | 50,0 |
| 3 | Aloe Ferox* | 0,5 |
| 4 | Rheopearl KL2 (Dextrin palmitate) | 10,0 |
| 5 | Lanolin | 5,0 |

| | | |
|---|---|---|
| *Aloe Ferox degree of concentration 1:1 | | |

**2.33**

| Item number | Ingredient | % |
|---|---|---|
| 1 | Triglycerides of caprinic and caprilic acid | 24,5 |
| 2 | Sage oil | 10,0 |
| 3 | Mint oil | 50,0 |
| 4 | Aloe Ferox* | 0,5 |
| 5 | Rheopearl KL2 (Dextrin palmitate) | 10,0 |
| 6 | Lanolin | 5,0 |

| | | |
|---|---|---|
| *Aloe Ferox degree of concentration 1:1 | | |

**2.34**

| Item number | Ingredient | % |
|---|---|---|
| 1 | Triglycerides of caprinic and caprilic acid | 9,5 |
| 2 | Sage oil | 25,0 |
| 3 | Mint oil | 50,0 |
| 4 | Aloe Ferox* | 0,5 |
| 5 | Rheopearl KL2 (Dextrin palmitate) | 10,0 |
| 6 | Lanolin | 5,0 |

| | | |
|---|---|---|
| *Aloe Ferox degree of concentration 1:1 | | |

**2.35**

| Item number | Ingredient | % |
|---|---|---|
| 1 | Triglycerides of caprinic and caprilic acid | 9,5 |
| 2 | Sage oil | 5,0 |
| 3 | Mint oil | 50,0 |
| 4 | Camphor oil | 20,0 |
| 5 | Aloe Ferox* | 0,5 |
| 6 | Rheopearl KL2 (Dextrin palmitate) | 10,0 |
| 7 | Lanolin | 5,0 |

| | | |
|---|---|---|
| *Aloe Ferox degree of concentration 1:1 | | |

**2.36**

| Item number | Ingredient | % |
|---|---|---|
| 1 | Triglycerides of caprinic and caprilic acid | 9,5 |
| 2 | Sage oil | 5,0 |
| 3 | Mint oil | 50,0 |
| 4 | Fennel oil | 20,0 |
| 5 | Aloe Ferox* | 0,5 |
| 6 | Rheopearl KL2 (Dextrin palmitate) | 10,0 |
| 7 | Lanolin | 5,0 |

| | | |
|---|---|---|
| *Aloe Ferox degree of concentration 1:1 | | |

**2.37**

| Item number | Ingredient | % |
|---|---|---|
| 1 | Triglycerides of caprinic and caprilic acid | 19,5 |
| 2 | Sage oil | 5,0 |
| 3 | Mint oil | 50,0 |
| 4 | Lavender oil | 10,0 |
| 5 | Aloe Ferox* | 0,5 |
| 6 | Rheopearl KL2 (Dextrin palmitate) | 10,0 |
| 7 | Lanolin | 5,0 |

| | | |
|---|---|---|
| *Aloe Ferox degree of concentration 1:1 | | |

## Claims

1. A pharmaceutical composition containing as active biocidal substances sage (*Salvia* sp.) essential oil and aloe *(Aloe* sp.) for use in a method of treatment and prophylaxis of inflammations caused by Demodex.

2. The composition for use according to claim 1, wherein sage essential oil is natural sage essential oil, such as *Salvia hispanica* oil, common sage *Salvia officinalis* oil or other species including *Salvia triloba* (*fruticosa*)*, Salvia lavandulifolia* and *Salvia sclarea.*

3. The composition for use according to claim 1 or 2, wherein the inflammation is inflammation around the eyes, in particular marginal blepharitis.

4. The composition for use according to any of claims 1 to 3, wherein aloe is selected from the group including *Aloe vera, Aloe ferox* and *Aloe arborescens* Mill.

## Patentansprüche

1. Pharmazeutische Zusammensetzung, enthaltend als aktive biozide Substanzen ätherisches Salbei- *(Salvia* sp.) Öl und Aloe (*Aloe* sp.) zur Anwendung in einer Behandlungsmethode und zur Prophylaxe gegen durch Demodex verursachte Entzündungen.

2. Zusammensetzung zur Anwendung nach Anspruch 1, bei der das ätherische Salbeiöl ein natürliches ätherisches Salbeiöl ist, wie zum Beispiel Chiasamen-Öl [*Salvia hispanica* Ö/], Öl aus echtem Salbei [*Salvia officinalis*] oder aus anderen Arten einschließlich *Salvia triloba* (*fruticosa*)*, Salvia lavandulifolia* und *Salvia sclarea.*

3. Zusammensetzung zur Anwendung nach Anspruch 1 oder 2, bei der die Entzündung eine Entzündung um die Augen herum ist, insbesondere marginale Blepharitis.

4. Zusammensetzung zur Anwendung nach einem der Ansprüche 1 bis 3, bei der Aloe aus der Gruppe ausgewählt wird, die *Aloe vera, Aloe ferox* und *Aloe arborescens Mill* umfasst.

## Revendications

1. Composition pharmaceutique contenant en tant que substance biocidique active de l'huile essentielle de sauge *(Salvia* sp.) et de l'aloès (*Aloe* sp.) pour l'utilisation dans une méthode de traitement et la prophylaxie d'inflammations causées par Demodex.

2. Composition pour l'utilisation selon la revendication 1, dans laquelle l'huile essentielle de sauge est l'huile essentielle de sauge naturelle, telle que l'huile de *Salvia hispanica,* l'huile de *Salvia officinalis* de sauge commune ou d'autres espèces incluant *Slavia triloba* (*fruticosa*)*, Salvia lavandulifolia* et *Salvia sclarea.*

3. Composition pour l'utilisation selon les revendications 1 ou 2, dans laquelle l'inflammation est une inflammation autour des yeux, en particulier la blépharite marginale.

4. Composition pour l'utilisation selon les revendications 1 à 3, dans laquelle l'aloès est choisi parmi le groupe incluant *Aloe vera, Aloe ferox* et *Aloe arborescens* Mill.
